# EUROPÄISCHE PATENTSCHRIFT

(11) **EP 2 776 806 B1**
(45) Veröffentlichungstag und Bekanntmachung des Hinweises auf die Patenterteilung: **12.04.2017**
(21) Anmeldenummer: 12772963.0
(22) Anmeldetag: 17.10.2012
(51) Int. Cl.: G01L 21/32, G01L 21/34, A61N 5/10, H01J 41/02, H05H 1/00, H05H 7/00

(54) **IONISATIONSMANOMETER**
IONIZATION MANOMETER
MANOMÈTRE À IONISATION

(30) Priorität: 07.11.2011 DE 102011055084
(43) Veröffentlichungstag der Anmeldung: 17.09.2014
(73) Patentinhaber: GSI Helmholtzzentrum für Schwerionenforschung GmbH, 64291 Darmstadt (DE)
(72) Erfinder: KOLLMUS, Holger, 60599 Frankfurt (DE)
(74) Vertreter: Blumbach Zinngrebe
(86) Internationale Anmeldenummer: PCT/EP2012/070561
(87) Internationale Veröffentlichungsnummer: WO 2013/068216

(56) Entgegenhaltungen:
- EP-A1- 1 519 402
- US-B1- 6 476 612
- SCHRAM B L ET AL: "Ionization cross sections for electrons (0.6-20 keV) in noble and diatomic gases", PHYSICA, ELSEVIER, AMSTERDAM, NL, Bd. 31, Nr. 1, 1. Januar 1965 (1965-01-01) , Seiten 94-112, XP024426779, ISSN: 0031-8914, DOI: 10.1016/0031-8914(65)90109-6 [gefunden am 1965-01-01]

## Beschreibung

Die Erfindung betrifft eine Messvorrichtung, die zumindest eine Elektronenaufnahmevorrichtung und zumindest eine lonenaufnahmevorrichtung aufweist. Weiterhin betrifft die Erfindung eine Niederdruckeinrichtung, insbesondere eine Teilchenbeschleunigereinrichtung und/oder eine Teilchenstrahlführungseinrichtung.

Typische Ionisationsmanometer sind aus EP1519402 und US6476612 bekannt.

Insbesondere für den Betrieb, die Ansteuerung und die Überwachung technischer Anlagen ist die Messung einer zum Teil großen Anzahl unterschiedlichster technischer Betriebsparameter erforderlich. Eine derartige Messung von unterschiedlichsten physikalischen Parametern kann sich natürlich auch in anderen Zusammenhängen als sinnvoll/erforderlich erweisen. Je nach konkret zu ermittelndem physikalischen Parameter, der dabei erforderlichen Genauigkeit sowie dem Größenbereich, in dem die Messung erfolgt, ist es dabei erforderlich unterschiedliche Messprinzipien und/oder unterschiedliche Messsysteme zu verwenden.

Ein Beispiel unter vielen ist dabei die Messung von Drücken, insbesondere die Messung von Drücken gasförmiger Substanzen. Das bekannteste, in diesem Zusammenhang verwendete Messprinzip, beruht dabei darauf, dass der Druck über die Kraft gemessen wird, die der Druck auf eine bestimmte Fläche ausübt. Bekannt sind in diesem Zusammenhang beispielsweise Luftdruck-Barometer, bei denen der umgebende Luftdruck im Verhältnis zum in einer verformbaren, geschlossenen Dose befindlichen Gasdruck (oftmals ein Vakuum), über die mechanische Verformung der Dose gemessen wird. Auch Druckmessgeräte, welche beispielsweise den Druck von technischen Gasen (beispielsweise Gasdruck in einer zylindrischen Vorratsflasche, Gasdrücke bei technischen Anlagen und dergleichen) und/oder von Dampf (beispielsweise bei Kraftwerken und dergleichen) messen, beruhen auf diesem Messprinzip. Druckmessgeräte, bei denen "technisch erzeugte" Drücke gemessen werden, werden dabei üblicherweise als Manometer bezeichnet.

Eine Messung von Drücken durch Messung einer Kraftausübung auf eine Fläche ist jedoch nur in bestimmten Druckbereichen möglich. Müssen dagegen beispielsweise extrem hohe Drücke oder umgekehrt extrem niedrige Drücke gemessen werden, so ist es oftmals erforderlich, auf anders geartete Messprinzipien zurückgreifen zu müssen. Entsprechendes gilt auch für Messungen in aggressiven Umgebungen, bei extrem hohen oder niedrigen Temperaturen und dergleichen.

Ein Beispiel hierfür ist die Messung extrem niedriger Drücke bei technischen Anlagen, bei denen Bereiche mit extrem niedrigen Gasdrücken (beispielsweise Feinvakuumbereich (FV), Hochvakuumbereich (HV), Ultrahochvakuumbereich (UHV), Extremhochvakuumbereich (XHV)) vorhanden sind. Hier liefern Manometer mit einer "klassischen Bauweise" in aller Regel keine brauchbaren Ergebnisse. Weiterhin können "klassische" Manometer auch deshalb ungeeignet sein, weil speziell im Ultrahochvakuumbereich (und bei noch niedrigeren Drücken) die zu verwendenden Gerätschaften bestimmte Randbedingungen erfüllen müssen, die von "klassischen" Manometern oftmals nicht erfüllt werden können. Beispielhaft ist hierbei die Fähigkeit zum Ausheizen zu nennen, die Fähigkeit bei extrem niedrigen Temperaturen betrieben werden zu können (zusätzliche Pumpwirkung durch kryostatisches Festfrieren von Restgas an den Behälterwänden), sowie eine besonders geringe Durchlässigkeit gegenüber Gasen (niedrige Gasdiffusion).

Aus diesen Gründen wird bei Anlagen, die bei derartig niedrigen Drücken (bzw. bei derartig guten Vakua) betrieben werden, in der Regel auf anders geartete Messprinzipien zurückgegriffen. Ein üblicherweise hierzu verwendetes Messprinzip liegt in Form von sogenannten "Ionisationsmanometern" vor. Ein typischer Aufbau derartiger Ionisationsmanometer sieht vor, dass mit Hilfe einer Glühkathode Elektronen in den freien Raum (in das Vakuum) abgegeben werden. Die so erzeugten freien Elektronen wandern zu einer (positiv geladenen) Elektrode (=Anode), wo sie der Anlage wieder entzogen werden. Aufgrund der zwischen der Glühkathode und der positiv geladenen Elektrode (=Anode) angelegten Spannung werden die Elektronen auf ihrem Weg auf eine zur Spannung korrespondierende Geschwindigkeit beschleunigt. Treffen die Elektronen dabei auf ein verbliebenes Restgasatom (beziehungsweise Restgasmolekül), so kann dies mit einer gewissen Wahrscheinlichkeit durch den Elektronenstoß zu einem Ion ionisiert werden. Das so erzeugte Ion (in aller Regel ein positiv geladenes Ion) wandert zu einer gesondert ausgebildeten Kathode, dem sogenannten Ionenkollektor. Der am lonenkollektor gemessene, durch die dort auftreffenden Ionen bewirkte Strom ist ein Maß für die im System verbliebene Restgaskonzentration - mit anderen Worten für den Gasdruck.

Derartige Ionisationsmanometer werden als kompakte Einheiten kommerziell gehandelt. Die entsprechenden Komponenten sind also in der Regel von einer einzelnen Gehäusung aufgenommen, in der die Komponenten des lonisationsmanometers angeordnet sind. Hier ist insbesondere die Glühkathode, die Elektronenanode und der Ionenkollektor, einschließlich der eigentlichen Messstrecke, in der der überwiegende Anteil der Wechselwirkung zwischen Elektronen und Restgasatomen/Restgasmolekülen (und damit die lonenerzeugung) erfolgt, zu nennen.

Obgleich derartige, kommerziell erhältliche Ionisationsmanometer durchaus funktionstüchtig sind, weisen diese jedoch auch Nachteile auf. Gerade bei zunehmend besser werdenden Vakua hat man nämlich das Problem, dass sich innerhalb eines fluidisch verbundenen Bereichs (also beispielsweise innerhalb einer Beam-Pipe bzw. innerhalb einer Beam-Pipe und einem damit über ein Flansch verbundenen Ionisationsmanometer) Druckunterschiede ausbilden können, die sich nicht oder erst nach vergleichsweise langer Zeit ausgleichen können. Mithin liegt nicht notwendigerweise im gesamten Bereich einer Beschleunigeranordnung der gleiche Restgasdruck vor, obgleich die Komponenten fluidisch miteinander verbunden sind. Dies kann begreiflicherweise zu Messfehlern führen, wenn ein kommerziell erhältliches Ionisationsmanometer "seitlich" über ein Flansch mit der Beam-Pipe verbunden ist. Insbesondere kann der Restgasdruck in der Beam-Pipe (insbesondere im Bereich des Teilchenstrahls) von dem Druck innerhalb der Messstrecke des Ionisationsmanometers abweichen.

Das genannte Problem, dass im Hochvakuumbereich unterschiedliche Drücke in fluidisch miteinander verbundenen Bereichen auftauchen können, wird zusätzlich verschärft, wenn die Temperaturen in unterschiedlichen Bereichen der Anlage (stark) unterschiedlich sind. Ist beispielsweise ein Ionisationsmanometer vom Glühkathodentyp (mit entsprechend heißen Oberflächen) an eine Beam-Pipe angeflanscht, und befindet sich im Bereich von bis zu einigen Metern Entfernung in der Beam-Pipe beispielsweise eine Kryopumpe (mit entsprechend niedrigen Temperaturen), so kommt es in diesen Bereichen zu vergleichsweise starken Druckunterschieden aufgrund der stark unterschiedlichen Oberflächentemperaturen. In diesem Zusammenhang spricht man auch von einer sogenannten "thermischen Transpiration".

Es ist leicht einsichtig, dass Messfehler ganz allgemein unerwünscht sind. Insbesondere beim Betrieb eines Teilchenstrahlbeschleunigers (oder sonstige Anlagen, bei denen sich zumindest Teile der Anlage im hohen Vakuumbereich befinden - insbesondere im Hochvakuumbereich, im Ultrahochvakuumbereich und/oder im Extremhochvakuumbereich) ist es darüber hinaus besonders wünschenswert Messungen speziell in solchen Bereichen durchführen zu können, in denen eine besondere Relevanz in Bezug auf die Anlage vorliegt. Dabei kann es sich beispielsweise bei Teilchenstrahlbeschleunigern um den Bereich der Beam-Line handeln, also den Bereich, in dem der Teilchenstrahl umläuft (bzw. die Teilchenstrahl-Pakete bzw. Teilchenstrahl-Bunches). Denn in diesem Bereich führt eine Wechselwirkung (insbesondere ein Stoß) zwischen Teilchenstrahl-Teilchen und Restgasatomen/Restgasmolekülen zu einer Abnahme der Anzahl der umlaufenden Teilchen, und damit zu einer Schwächung des Teilchenstrahls. Dementsprechend wird eine Messung, die im Bereich des Teilchenstrahls selbst vorgenommen wird, besonders sinnvolle Aussagen ermöglichen, die mit kommerziell erhältlichen Ionisationsmanometern oftmals nicht möglich sind.

Obgleich vorliegend in der Regel auf eine Verwendung der Messvorrichtung im Zusammenhang mit Teilchenbeschleunigern Bezug genommen wird, kann die Messvorrichtung selbstverständlich auch in anderen Gebieten vorteilhaft verwendet werden, bei denen besonders niedrige Gasdrücke gemessen werden müssen.
Wie erläutert besteht nach wie vor ein großes Interesse an Messvorrichtungen, mit denen insbesondere besonders niedrige Drücke im Vergleich zu bekannten Messvorrichtungen besser, gezielter und unproblematischer gemessen werden können.

Die vorliegende Erfindung löst dieses Problem. Der Gegenstand der Erfindung wird durch den Patentanspruch 1 definiert.

Es wird vorgeschlagen, eine Messvorrichtung, die zumindest eine Elektronenaufnahmevorrichtung und zumindest eine Ionenaufnahmevorrichtung aufweist, derart mit zumindest einer Messstreckeneinrichtung zu versehen, dass diese zumindest eine Messstreckeneinrichtung gesondert von der zumindest einen Elektronenaufnahmevorrichtung ausgebildet ist. Dadurch ist es möglich, dass bei der Messvorrichtung die eigentliche Messstrecke, also insbesondere der Bereich, in dem der überwiegende Anteil der Wechselwirkungen zwischen ionisierenden Teilchen (in der Regel Elektronen) und (Rest-) Gasatomen / (Rest-) Gasmolekülen stattfindet, im Wesentlichen unabhängig von den sonstigen Teilen der Messvorrichtung (insbesondere räumlich unabhängig) gewählt werden kann, und insbesondere unabhängig von der zumindest einen Elektronenaufnahmevorrichtung und/oder der zumindest einen Ionenaufnahmevorrichtung gewählt werden kann. Dies ermöglicht es insbesondere, dass bei der Größe, der Anordnung, der Ausbildungsform, dem Messprinzip, den sonstigen (insbesondere zusätzlichen) Eigenschaften und dergleichen eine deutlich geringere Rücksichtnahme auf den eigentlichen Messort bzw. sogar eine im Wesentlichen vom eigentlichen Messort unabhängige Ausbildung möglich wird. Die Ausbildung der Messvorrichtung (insbesondere die Anordnung der zumindest einen Messstreckeneinrichtung und/oder der zumindest einen Ionenaufnahmevorrichtung) erfolgt derart, dass im Wesentlichen ausschließlich Ionen, welche im Bereich der Messstreckeneinrichtung erzeugt werden, messtechnisch erfasst werden. Dies kann insbesondere auch bedeuten, dass die eigentliche Messstrecke (der Messort) der Messvorrichtung deutlich weniger von den sonstigen Teilen der Messvorrichtung beeinflusst ist bzw. sonstige Teile der Messvorrichtung geringere Anforderungen/Bedingungen an die Messstreckeneinrichtung stellen. Insbesondere ist es möglich, dass im Bereich der Messstreckenvorrichtung zusätzliche Geräte vorgesehen werden können (was sonst oftmals bereits aufgrund von Platzproblemen problematisch bis unmöglich ist) und/oder Funktionen und Eigenschaften realisiert werden können, die sonst nicht bzw. bestenfalls problematisch erzielt werden können. Möglich ist es dadurch insbesondere, dass beispielsweise ein (Rest-) Gasdruck in besonders interessierenden Bereichen gemessen werden kann, wie beispielsweise im Bereich der Teilchenflugbahn bei einem Teilchenbeschleuniger, im Bereich von kryostatischen Anlagen, von Messsensoren, von Experimentiervorrichtungen (beispielsweise Target-Vorrichtungen) und dergleichen. Bei der Elektronenaufnahmevorrichtung kann es sich um eine im Prinzip beliebige Form einer Elektronenaufnahmevorrichtung handeln. Beispielsweise kann es sich um eine einfache Elektronenkathode, eine Bayard-Albert-Drahtelektrode, einen Zylinderkäfig mit einer Faraday-Cup und dergleichen handeln. Möglich ist es aber auch, dass es sich zusätzlich oder alternativ um ein einfaches, gegebenenfalls vorgespanntes Blech und/oder um einen elektrisch leitfähigen (insbesondere metallischen), vorzugsweise flächigen (Oberflächen-) Bereich (beispielsweise eine Gehäusewandung wie das Rohr einer Beam-Pipe) handelt. Eine wesentliche Aufgabe der Elektronenaufnahmevorrichtung kann darin bestehen, die Ausbildung eines geladenen Raumbereichs durch eine Ansammlung von "nicht abgesaugten" Elektronen zu vermeiden. Ein derartiger Bereich könnte beispielsweise bereits erzeugte Ionen "zerstören", bevor diese gemessen werden können. Vorteilhaft ist es, wenn die Elektronenaufnahmevorrichtung derart ausgebildet und eingerichtet ist, dass es zu keiner (bzw. einer nur vergleichsweise geringen) Verfälschung der Messung durch Sekundärteilchenemission kommt. Selbstverständlich kann im Zusammenhang mit der Elektronenaufnahmevorrichtung auch an messverstärkende Bauformen und/oder messverstärkende Prinzipien zu denken sein. Insbesondere ist an eine Art "Photomultiplier" zu denken, bei dem einfallende Elektronen lawinenartig verstärkt werden. Ein besonders großer Vorteil bei einer Messung der Menge an aufgefangenen Elektronen (Messung erfolgt in der Regel durch den sogenannten Elektronenstrom bzw. Kathodenstrom) ist, dass hierdurch ein besonders vorteilhaftes und genaues Eichnormal geliefert werden kann, mithilfe dessen der Ionenstrom normiert werden kann. Unter einer Ionenaufnahmevorrichtung ist ebenfalls grundsätzlich an jede denkbare Form von Ionenaufnahmevorrichtung zu denken, wie insbesondere an Drahtelektroden, an flächige Elektroden und/oder an Gitterelektroden. Gegebenenfalls kommen auch Ionenaufnahmevorrichtungen mit messverstärkenden Eigenschaften und/oder messverstärkenden Prinzipien und/oder solche, die mit messverstärkenden Vorrichtungen kombiniert sind, in Betracht. Im Zusammenhang mit messverstärkenden Vorrichtungen (insbesondere für Ionen und/oder Elektronen) ist insbesondere an sogenannte Micro channel plates" und/oder "Channeltrons" zu denken. Derartige Bauteile dienen in der Regel als eine Art Photomultiplier, wobei üblicherweise jedoch nicht Photonen, sondern Ionen und/oder Elektronen in eine Elektronenlawine "umgewandelt" werden. Ein weiterer Vorteil der vorgeschlagenen Messvorrichtung ist, dass es durch die unabhängige Ausbildung von Messstreckeneinrichtung, Ionenaufnahmevorrichtung und Elektronenaufnahmevorrichtung möglich ist, Druckmessungen in beispielsweise besonders schwer zu erreichenden Bereichen ("verbaute Bereiche") durchführen zu können. Dies ist verständlicherweise von Vorteil.

Vorteilhaft kann es sein, wenn die zumindest eine Ionenaufnahmevorrichtung zumindest teilweise und/oder zumindest zeitweise im Bereich und/oder benachbart zu der zumindest einen Messstreckeneinrichtung angeordnet ist. Erste Versuche haben gezeigt, dass hierdurch eine besonders große Empfindlichkeit bzw. Genauigkeit der Messvorrichtung realisiert werden kann. Basierend auf ersten Annahmen liegt dies daran, dass die durch einen Elektronenstoß erzeugten Ionen eine typischerweise nur geringe kinetische Energie im Millielektronenvoltbereich (meV) aufweisen. Dadurch können diese einerseits nur vergleichsweise geringe Distanzen zurücklegen, insbesondere ohne dass diese von in der Nähe befindlichen Metallflächen "angesaugt" und "vernichtet" werden. Andererseits können auch Zeitverzögerungseffekte durch die vergleichsweise langsam fliegenden Restgasionen vermieden werden. Vorzugsweise ist die zumindest eine Ionenaufnahmevorrichtung so angeordnet, dass sie gegebenenfalls zusätzlich angeordnete Geräte und/oder zusätzliche Eigenschaften der Messvorrichtung bzw. der Messstreckeneinrichtung nicht bzw. nur in geringem Ausmaß behindert.

Weiterhin wird vorgeschlagen, dass die zumindest eine Messstreckeneinrichtung als mit einer weiteren Aufgabe betraute Kombinationseinrichtung ausgebildet ist, und zumindest eine Durchgangseinrichtung, sowie insbesondere zumindest eine Transporteinrichtung, zumindest eine zusätzliche Messeinrichtung, zumindest eine zusätzliche Sensoreinrichtung, zumindest eine Experimentiereinrichtung, zumindest eine Beeinflussungseinrichtung und/oder zumindest eine Pumpeinrichtung aufweist und/oder zumindest teilweise als eine solche ausgebildet ist. Gerade an solchen Stellen, an denen eine zusätzliche Aufgabe zusätzlich zur Messung (insbesondere Druckmessung) erfolgt, ist der ermittelte Messwert in aller Regel besonders aussagekräftig bzw. relevant. Dementsprechend ist es mit der vorgeschlagenen Weiterbildung der Messvorrichtung möglich, eine Messung "unmittelbar am interessierenden Teil" bzw. "unmittelbar im interessierenden Bereich" durchzuführen. Als derartige zusätzliche Aufgaben ist insbesondere an einen Durchgang eines Teilchenstrahls, sonstiger Materie oder sonstiger Strahlung zu denken (Durchgangseinrichtung), an einen Transport von größeren Mengen Materie (Transporteinrichtung, wie insbesondere Pumpeinrichtung und dergleichen), an eine Messung zusätzlicher Parameter (zusätzliche Messeinrichtung), an eine Registrierung sonstiger Eigenschaften (zusätzliche Sensoreinrichtung) oder an ein Experiment, wie beispielsweise zu bestrahlende Materie (Experimentiereinrichtung) . Weiterhin ist an eine Beeinflussung, beispielsweise eines Teilchenstrahls durch einen Magnet (Beeinflussungseinrichtung) zu denken. Dabei ist es nicht notwendig, dass die gesamte zusätzliche Aufgabe im Bereich der Messstreckeneinrichtung durchgeführt wird bzw. die gesamte Messstreckeneinrichtung im Bereich einer zusätzlichen Aufgabe angeordnet ist. Vielmehr ist auch ein "teilweiser Überlapp" denkbar und oftmals sinnvoll.

Weiterhin wird vorgeschlagen, dass die zumindest eine Elektronenaufnahmevorrichtung, die zumindest eine Ionenaufnahmevorrichtung und/oder die zumindest eine Messstreckeneinrichtung zumindest zeitweise und/oder zumindest bereichsweise voneinander beabstandet angeordnet sind, insbesondere räumlich voneinander getrennt ausgebildet und angeordnet sind. Bei einer derartigen Ausbildung der Messvorrichtung können die einzelnen Komponenten nochmals besser und individueller an ihre jeweilige Aufgabe angepasst werden, ohne dass "Einflussfaktoren" von (den) anderen Komponenten in relevantem Ausmaß zu berücksichtigen sind bzw. Einflüsse in relevantem Ausmaß erfolgen. Insbesondere ist in diesem Zusammenhang auch an eine Art "Baukastenprinzip" zu denken, bei dem aus einem vorgegebenen Satz an einzelnen Einrichtungen (insbesondere Elektronenaufnahmevorrichtungen, Ionenaufnahmevorrichtungen und Messstreckeneinrichtungen, gegebenenfalls aber auch weitere Komponenten) das jeweils vorteilhafteste Teil aus dem jeweiligen Satz verwendet wird und diese Einzelkomponenten zu einer besonders geeigneten Messvorrichtung kombiniert werden. Dadurch kann die Flexibilität der Messvorrichtung oftmals deutlich erhöht werden, ohne dass übermäßige Kosten und/oder Lagerhaltungsprobleme auftreten müssen.

Die Messvorrichtung ist als Druckmessvorrichtung ausgebildet, insbesondere als Niederdruckmessvorrichtung, bevorzugt als Druckmessvorrichtung für Grobvakuum, Feinvakuum, Hochvakuum, Ultrahochvakuum, Extremhochvakuum und/oder für Tieftemperaturumgebungen ausgebildet ist Beispielsweise kann es sich um eine typischerweise als Ionisationsmanometer bezeichnete Vorrichtung zur Messung insbesondere niedriger Drücke handeln. Insbesondere ist es möglich, dass die Messvorrichtung als Niederdruckmessvorrichtung, bevorzugt als Druckmessvorrichtung für Grobvakuum (GV; typischerweise zwischen 3x10⁴ Pa und 1x10² Pa), Feinvakuum (FV; typischerweise zwischen 1x10² Pa und 1x10⁻¹ Pa), Hochvakuum (HV; typischerweise zwischen 1x10⁻¹ Pa und 1x10⁻⁵ Pa), Ultrahochvakuum (UHV; typischerweise zwischen 1x10⁻⁵ Pa und 1x10⁻¹⁰ Pa) sowie Extremhochvakuum (XHV; typischerweise weniger als 1x10⁻¹⁰ Pa) und/oder für Tieftemperaturumgebungen ausgebildet ist (insbesondere Temperaturen bei oder unterhalb der Temperatur von flüssigem Stickstoff bzw. flüssigem Helium). Gerade für derartige Messumgebungen hat sich das Messprinzip der Ionisation von (Rest-) Gasatomen / (Rest-) Gasmolekülen als besonders vorteilhaft erwiesen. Mit der vorgeschlagenen Ausbildung der Messvorrichtung ergeben sich besondere Vorzüge (die insbesondere bereits beschrieben wurden bzw. noch beschrieben werden). Insbesondere ist bei derart niedrigen Drücken die Verwendung anderer Messverfahren, insbesondere von "klassischen" Messverfahren, bei denen die Krafteinwirkung auf eine definierte Fläche gemessen wird, in aller Regel nicht mehr möglich bzw. nicht ausreichend genau. Insofern sind andere Messprinzipien anzuwenden, von denen sich das vorbeschriebene Messprinzip als besonders geeignet erweist.

Erste Versuche haben ergeben, dass es besonders vorteilhaft ist, wenn die Messvorrichtung derart ausgebildet und eingerichtet ist, dass in zumindest einem Bereich der Messstreckeneinrichtung zumindest ein Teil der Elektronen zumindest zeitweise eine Teilchenenergie zwischen 20 eV und 1 keV, bevorzugt zwischen 50 eV und 500 eV, besonders bevorzugt zwischen 80 eV und 300 eV, insbesondere zwischen 100 eV und 200 eV aufweist und/oder dass in zumindest einem Bereich der Messstreckeneinrichtung zumindest zeitweise ein Elektronenstrom mit einer Stromstärke von ≥ 0,2 mA, bevorzugt von ≥ 0,5 mA, besonders bevorzugt von ≥ 0,8 mA, insbesondere von ≥ 1 mA vorhanden ist. Dies gilt insbesondere dann, wenn die Ionisierung der (Rest-) Gasatome bzw. (Rest-) Gasmoleküle durch Interaktion mit Elektronen, insbesondere mit speziell für diesen Zweck erzeugten Elektronen, erfolgt. Unter der zumindest einen Messstreckeneinrichtung ist insbesondere ein Bereich zu verstehen, in dem ein üblicherweise besonders großer Anteil der Messung erfolgt (also der "eigentliche" Messort). Dies schließt selbstverständlich nicht aus, dass zu einem gewissen Anteil auch außerhalb dieses Bereichs eine gewisse Messung (beispielsweise durch Ionisierung von Restgasatomen/Restgasmolekülen) erfolgt. Insbesondere kann es sich bei der Messstreckeneinrichtung um einen Bereich handeln, in dem ein vorzugsweise besonders großer Anteil an Interaktion zwischen Restgasatomen (Restgasmolekülen) und Elektronen stattfindet. Erste Versuche haben ergeben, dass die genannten Wertebereiche für die Elektronenenergien im vorliegenden Zusammenhang besonders vorteilhaft sind. Einerseits führen die vorab vorgeschlagenen Energiebereiche mit einer in Relation gesehenen besonders hohen Wahrscheinlichkeit zur Ionisierung eines von einem Elektron des Elektronenstrahls getroffenen Restgasatoms bzw. Restgasmoleküls. Gleichzeitig ist die Energie der Elektronen jedoch noch ausreichend groß, dass diese ausreichend "richtungsstabil" weiterfliegen, wenn diese nicht mit Restgasmolekülen bzw. Restgasatomen interagieren. Oftmals fliegen jedoch auch bereits interagierte Elektronen ausreichend "richtungsstabil" weiter, insbesondere dann, wenn diese vor der Interaktion eine entsprechend geeignete Energie aufwiesen. Dadurch können die nicht interagierten Elektronen in einem relativ eng begrenzten räumlichen Bereich "abgefangen" werden und insbesondere auch von den erzeugten Restgasionen separiert werden. Insbesondere ist darauf hinzuweisen, dass die Restgasionen typischerweise kinetische Energien im Millielektronenvolt-Bereich (meV-Bereich) haben. Dementsprechend sind sehr kleine elektrische Felder (typischerweise im Bereich von 1 V/cm bis 10 V/cm) ausreichend, um diese vergleichsweise schnell und mit einer geringen Verlustwahrscheinlichkeit zu einer geeigneten Ionenfängerelektrode zu "ziehen". Die genannten elektrischen (lonensammel-) Feldstärken sind jedoch so niedrig, dass sie Elektronen in den genannten Energiebereichen lediglich in einem hinreichend kleinen Ausmaß beeinflussen. Insgesamt können sich dadurch besonders vorteilhafte Messungen ergeben. Bei Verwendung der vorgeschlagenen Stromstärke ist es darüber hinaus möglich, dass es mit einer ausreichenden Wahrscheinlichkeit zu einer ausreichenden Anzahl von Wechselwirkungen pro Zeiteinheit kommt, derart, dass ein "vernünftig großes" Signal, insbesondere an der Ionenaufnahmevorrichtung (insbesondere lonenauffangkathode/lonenkollektor) erzeugt wird. Insbesondere ist der gemessene Ionenstrom dann üblicherweise gut messtechnisch erfassbar und die dort auftretenden statistischen Schwankungen halten sich üblicherweise in vertretbaren Grenzen. Wird zusätzlich der "hindurchgegangene" Elektronenstrom gemessen, so steht ein besonders geeigneter Referenzwert zur Normierung des Ionenstroms zur Verfügung. Insbesondere bei Elektronenfreisetzungsvorrichtungen, die ein gewisses Schwankungsverhalten zeigen, kann sich dies als besonders vorteilhaft erweisen.

Weiterhin wird vorgeschlagen, dass bei der Messvorrichtung die zumindest eine Ionenaufnahmevorrichtung als lonenabsaugvorrichtung und/oder als vorzugsweise flächige Ionenkollektoreinrichtung ausgebildet ist und/oder zumindest bereichsweise zumindest eine Teilchenvervielfachungs-Verstärkungseinrichtung aufweist und/oder dadurch, dass zumindest eine Elektronenaufnahmevorrichtung zumindest teilweise als Auffangelektrodeneinrichtung ausgebildet ist. Unter Ionenabsaugvorrichtung kann insbesondere eine Vorrichtung verstanden werden, die "aktiv Ionen ansaugt". Dies kann beispielsweise durch Anlegen geeigneter elektrischer Felder (insbesondere hinsichtlich Richtung und/oder Stärke) erfolgen. Dies stellt typischerweise einen Unterschied zu Aufnahmevorrichtungen dar, bei denen die Ionen lediglich "zufällig" und statistisch auf die betreffende Aufnahmevorrichtung treffen, obgleich grundsätzlich auch solche Aufnahmevorrichtungen geeignet sind. Eine vorzugsweise flächige Ausbildung kann den Auffangquerschnitt und damit die insgesamt aufgenommene Anzahl an Ionen (zusätzlich) erhöhen. Besonders vorzugsweise wird die Geometrie und Größe der Ionenaufnahmevorrichtung derart gewählt, dass (weitgehend) alle Ionen aufgefangen werden. Dadurch kann insbesondere der Ionenstrom erhöht werden. Typischerweise lassen sich dadurch genauere und/oder statistisch weniger fluktuierende Messwerte erzielen. Eine Teilchenvervielfachungs-Verstärkungseinrichtung kann im Zusammenhang mit Ionen insbesondere in Form von bereits erwähnten "Micro channel plates" und/oder "Channeltrons" erfolgen. Für die Elektronenaufnahmevorrichtung kann das im Zusammenhang mit der Ionenaufnahmevorrichtung gesagte in Analogie gelten.

Weiterhin kann es vorteilhaft sein, wenn bei der Messvorrichtung zumindest eine Elektronenfreisetzungsvorrichtung vorgesehen ist, welche zumindest zeitweise und/oder zumindest bereichsweise von der zumindest einen Elektronenaufnahmevorrichtung und/oder von der zumindest einen Messstreckeneinrichtung und/oder von der zumindest einen Ionenauffangeinrichtung beabstandet angeordnet ist, insbesondere räumlich getrennt ausgebildet und angeordnet ist und/oder dadurch, dass die zumindest eine Elektronenfreisetzungsvorrichtung zumindest teilweise als Glühkathodeneinrichtung, als Kaltkathodeneinrichtung und/oder als Elektronenkanoneneinrichtung ausgebildet ist. Wenn zumindest eine Elektronenfreisetzungsvorrichtung vorgesehen ist, kann auf einfache Weise für eine ausreichend große Menge an Elektronen gesorgt werden, so dass es schlussendlich auch zu einer ausreichend großen Anzahl an erzeugten Restgasionen (nach Interaktion eines Elektrons mit einem Restgasmolekül bzw. einem Restgasatom) kommt. Weiterhin können die von der Elektronenfreisetzungsvorrichtung freigesetzten Elektronen vorteilhafte Eigenschaften aufweisen, wie beispielsweise eine vorteilhafte Energie, eine vorteilhafte Bündelung (insbesondere strahlartig), einen vorteilhaften Elektronenfluss und/oder eine vorteilhafte Energiedispersion. Vorteilhaft ist in diesem Zusammenhang, wenn vergleichsweise stark emittierende Elektronenfreisetzungsvorrichtungen verwendet werden, die darüber hinaus bevorzugt in einem definierten Energiespektrum Elektronen freisetzen (gegebenenfalls auch nach Filterung) und/oder einen vergleichsweise konstanten Elektronenstrom freisetzen. Wird die zumindest eine Elektronenfreisetzungsvorrichtung separat ausgebildet, so kann diese besser auf ihren jeweiligen Zweck hin optimiert werden, ohne andere Vorrichtungen (insbesondere die genannten) nachteilig zu beeinflussen. Die genannten Ausbildungsmöglichkeiten der Elektronenfreisetzungsvorrichtung haben sich in ersten Versuchen als besonders vorteilhaft erwiesen. Insbesondere ist darauf hinzuweisen, dass insbesondere bei einer getrennten Ausbildung der Elektronenfreisetzungsvorrichtung auch eine Glühkathodeneinrichtung im Zusammenhang mit kryostatischen Systemen verwendet werden kann. Durch eine getrennte Ausbildung können nämlich die thermischen Energieeinträge durch die Glühkathodeneinrichtung in der Regel in einem vertretbaren Rahmen gehalten werden. Insbesondere ist es möglich unter Verwendung eines Kalt-Warm-Übergangs die Glühkathode in einer Raumtemperaturumgebung, in einer mit flüssigem Stickstoff gekühlten Umgebung und/oder in einer mit flüssigem Neon gekühlten Umgebung zu betreiben.

Insbesondere im vorab beschriebenen Zusammenhang (aber gegebenenfalls auch in anderen Zusammenhängen) kann es sich als vorteilhaft erweisen, wenn bei der Messvorrichtung die zumindest eine Messstreckeneinrichtung und/oder die zumindest eine Elektronenaufnahmevorrichtung und/oder die zumindest eine Ionenaufnahmevorrichtung und/oder die zumindest eine Elektronenfreisetzungsvorrichtung zumindest zeitweise und/oder zumindest bereichsweise durch zumindest eine thermische Isolationseinrichtung voneinander getrennt sind, insbesondere räumlich voneinander getrennt sind. Hierdurch können "thermische Überträge" besonders effektiv verhindert werden. Insbesondere eine Glühkathodeneinrichtung kann dann besonders geringe nachteilige thermische Effekte zur Folge haben.

Weiterhin wird vorgeschlagen, die Messvorrichtung mit zumindest einer Elektronenbeschleunigungseinrichtung, zumindest einer Elektronenfokussiereinrichtung, zumindest einer Elektronenablenkeinrichtung, zumindest einer Elektronenabbremseinrichtung, zumindest einer Elektronendefokussiereinrichtung, zumindest einer Streuelektronenabsaugeinrichtung und/oder zumindest einer lonenabsaugeinrichtung und/oder zumindest einer Bahnbeeinflussungseinrichtung auszubilden. Durch die genannten Einrichtungen (die sowohl einzeln, als auch in Kombination vorgesehen werden können, wobei es durchaus auch möglich ist, dass einzelne oder mehrere Einrichtungen auch mehrfach vorgesehen werden) ist es insbesondere möglich, dass die entsprechenden Teilchen (insbesondere geladene Teilchen wie Ionen und/oder Elektronen) in besonders geeigneter Weise auf die jeweils durchzuführende Messaufgabe angepasst werden können. Auch kann es dadurch ermöglich werden, dass die eigentliche Messstrecke so gelegt werden kann, dass eine Restgasdruckmessung in Bereichen möglich wird, die ansonsten bestenfalls nur mit Einschränkungen einer Messung zugänglich sind. Auch kann zusätzlich oder alternativ die Messgenauigkeit, die Messempfindlichkeit, die Selektivität gegenüber bestimmten Atomen (bzw. Molekülen) und dergleichen erhöht oder ganz im Gegenteil gezielt verringert werden. Bahnbeeinflussungseinrichtungen können insbesondere in Form von zumindest einer Magnetfelderzeugungsvorrichtung und/oder zumindest einer Elektrisches-Feld-Erzeugungsvorrichtung ausgebildet werden. Die zumindest eine Magnetfelderzeugungsvorrichtung bzw. die zumindest eine Elektrisches-Feld-Erzeugungsvorrichtung kann dabei zumindest teilweise als Teilchenführungsvorrichtung und/oder zumindest teilweise als Teilchenaufspiralungseinrichtung ausgebildet sein. Insbesondere ist es möglich, dass Ionen (insbesondere ionisierte Restgasmoleküle) durch die elektrischen und/oder magnetischen Felder in Richtung bestimmter Bereiche in bzw. benachbart zur Messstreckeneinrichtung gelenkt werden, wie insbesondere in Richtung zumindest einer Ionenaufnahmevorrichtung. Weiterhin ist es möglich, dass Elektronen auf bestimmte Bahnen gezwungen werden, insbesondere derart, dass sie einen längeren Weg zurücklegen und/oder dass sie dadurch Energie verlieren (um auf diese Weise beispielsweise die Wahrscheinlichkeit einer Interaktion bzw. eines Stoßes mit einem Restgasatom bzw. Restgasmolekül zu erhöhen).

Die Messvorrichtung ist vakuumdicht ausgeführt. Dies ist insbesondere dann von Vorteil, wenn mit der Messvorrichtung Vakua gemessen werden sollen bzw. die Messvorrichtung im Zusammenhang mit unter Vakuum betriebenen Anlagen bzw. Anlagenteilen verwendet werden soll.

Grundsätzlich ist es möglich, dass die Messvorrichtung, bzw. dass Teile der Messvorrichtung, als einteilige Vorrichtung ausgebildet ist (sind), die beispielsweise mit Hilfe eines einzigen Flanschs oder mithilfe mehrerer Flansche mit einer Vakuumapparatur verbunden werden kann (können) und die auch als separate, eigenständige Anordnung vorliegen kann. Ebenso ist es auch möglich, dass die Messvorrichtung mehrteilig ausgebildet ist, derart, dass beispielsweise zumindest eine Messstreckeneinrichtung und/oder zumindest eine Elektronenaufnahmevorrichtung als eigenständige, separate Untereinheit gehandhabt werden kann. Eine derartige getrennte Vorrichtung wird dann erst durch Zusammenfügen der Einzelkomponenten zu einer vollwertigen, vollständig funktionsfähigen Messvorrichtung. Insbesondere soll es möglich sein, dass für die Einzelkomponenten (insbesondere für die Messstreckeneinrichtung und/oder für die Elektronenaufnahmevorrichtung) jeweils alleine, als auch in deren (teilweiser) Kombination Schutz beansprucht werden kann. Darüber hinaus soll für einen "Bausatz", der zumindest eine Messstreckeneinrichtung sowie zumindest eine Elektronenaufnahmevorrichtung umfasst und zu einer Messvorrichtung vom vorgeschlagenen Typ zusammengefügt werden kann (gegebenenfalls unter Hinzufügung weiterer Bauelemente, wie beispielsweise einer Beam-Pipe, einer Elektronenfreisetzungsvorrichtung, einer Vakuumanlage usw.), Schutz beansprucht werden können. Im Übrigen ist es auch möglich, dass bereits ein derartiger "Bausatz" als Messvorrichtung im Sinne der vorab abgegebenen Beschreibung und/oder der folgenden Patentansprüche aufgefasst werden kann.

Weiterhin wird eine Niederdruckeinrichtung vorgeschlagen, die zumindest eine Messvorrichtung mit dem oben beschriebenen Aufbau aufweist. Bei der Niederdruckeinrichtung kann es sich insbesondere um eine Teilchenbeschleunigereinrichtung (beispielsweise Linearbeschleuniger, Synchotron, Speicherring, Zyklotron und dergleichen) und/oder um eine Teilchenstrahlführungseinrichtung (beispielsweise um ein Strahlrohr zum Transport eines Teilchenstrahls zu einer Experimentieranordnung, zu einem Behandlungsplatz oder zu einem Bestrahlungsplatz) und/oder um eine Ionenquelle (einschließlich der dazugehörigen Teilchentransportstrecken und dergleichen) handeln. Eine derartige Niederdruckeinrichtung weist die bereits vorab beschriebenen Vorteile und Eigenschaften der Messvorrichtung in analoger Weise auf. Auch ist es möglich, die Niederdruckeinrichtung im Sinne der vorab abgegebenen Beschreibung fortzuentwickeln.

Im Folgenden wird die Erfindung anhand vorteilhafter Ausführungsbeispiele und unter Bezugnahme auf die beigefügte Zeichnung näher erläutert. Es zeigen:
- Fig. 1:: ein erstes Ausführungsbeispiel für ein Ionisationsmanometer in einer schematischen Prinzipdarstellung;
- Fig. 2:: ein zweites Ausführungsbeispiel für ein Ionisationsmanometer in einer schematischen Prinzipdarstellung;
- Fig. 3:: ein drittes Ausführungsbeispiel für ein Ionisationsmanometer in einer schematischen Prinzipdarstellung;
- Fig. 4:: ein viertes Ausführungsbeispiel für ein Ionisationsmanometer in einer schematischen Prinzipdarstellung;
- Fig. 5:: ein fünftes Ausführungsbeispiel für ein Ionisationsmanometer in einer schematischen Prinzipdarstellung.

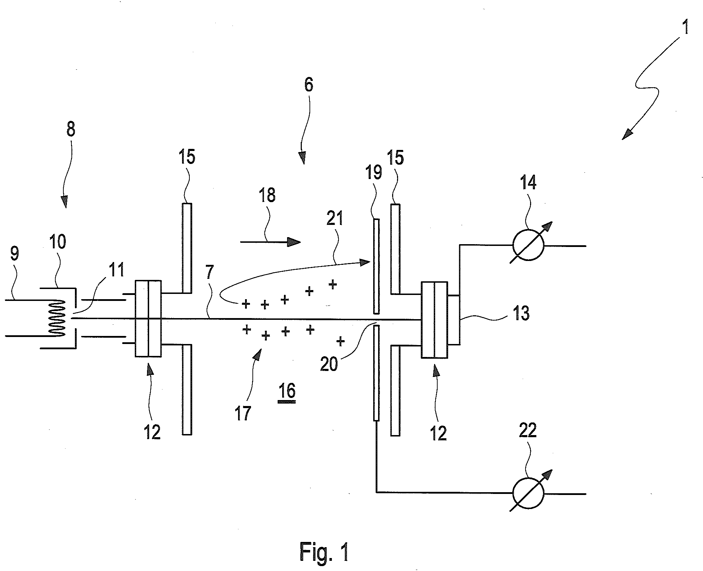

In Fig. 1 ist ein erstes Prinzipbeispiel für ein Ionisationsmanometer 1 dargestellt, mit dem direkt und unmittelbar der Restgasdruck in der UHV-Umgebung 16 einer Beam-Line 6 gemessen werden kann. Die Beam-Line 6 ist vorliegend nur als schematischer Ausschnitt dargestellt. In einem realen Ausführungsbeispiel würde diese beispielsweise bei einem Synchrotron eine in sich geschlossene, gekrümmte Bahn bilden, entlang derer eine große Anzahl von zusätzlichen Komponenten angebracht wäre (beispielsweise Extraktions- und Injektionsmagnete, Kickermagnete, Biegemagnete, Quadropolmagnete, Beschleunigungsstrecken, Vakuumpumpen und dergleichen). Typischerweise handelt es sich bei einer Beam-Line 6 um eine Röhre mit im wesentlichen kreisrunden Querschnitt, welche auf ein sehr hohes Vakuumniveau 16 evakuiert ist (typischerweise Ultrahochvakuumbereich 16 und besser).

Aufgrund der nur noch geringen Restgasdichte im Inneren der Beam-Line 6, versagen "klassische Druckmessverfahren" mit Druckdosen und dergleichen.

Aus diesem Grund ist ein Ionisationsmanometer 1 vorgesehen, welches von seinem Messprinzip her darauf beruht, dass ein Elektronenstrahl 7 durch das zu vermessende Gasvolumen gesendet wird. Dabei kommt es zu Wechselwirkungen (Stößen) zwischen den Elektronen des Elektronenstrahls 7 sowie den im zu vermessenden Volumen verbliebenen Restgasmolekülen bzw. Restgasatomen.

Beim vorliegend dargestellten Ausführungsbeispiel dient zur Erzeugung des Elektronenstahls 7 eine Elektronenquelle 8, die mit Hilfe einer Glühkathode 9 Elektronen freisetzt. Die von der Glühkathode 9 freigesetzten Elektronen weisen zunächst noch eine statistisch verteilte Energie- sowie Richtungsverteilung auf. Mit Hilfe einer Beschleunigungsanode 10, welche ein Durchgangsloch 11 mit geeigneter Größe aufweist, wird aus der von der Glühkathode 9 freigesetzten Elektronenwolke ein Elektronenstrahl 7 mit relativ homogener Teilchenenergie geformt.

Die Elektronenquelle 8 ist über eine Flanschverbindung 12 vakuumdicht mit der Beam-Line 6 verbunden. Der Elektronenstrahl 7 ist auf die gegenüberliegende Begrenzungswand 15 der Beam-Line 6 gerichtet.

Im "Zielgebiet" des Elektronenstrahls 7 ist - ebenfalls über eine vakuumdichte Flanschverbindung 12 - ein Elektronenkollektor 13 vorgesehen. Dieser fängt die "ungenutzten" Elektronen auf und misst darüber hinaus mit Hilfe eines Strommessgerätes 14 die Stärke des Elektronenstroms. Bei einer entsprechenden Energie der Elektronen können in der Regel auch die bereits "genutzten" Elektronen vom Elektronenkollektor 13 aufgefangen werden, da diese trotz der erfolgten Interaktion in der Regel ausreichend richtungsstabil weiterfliegen. Der Elektronenstrom kann als Vergleichsnormal in Relation zum gemessenen Ionenstrom 22 (vergleiche spätere Beschreibung) verwendet werden, um den Restgasdruck in der Beam-Line 6 bestimmen zu können.

Kommt es in der UHV-Umgebung 16 in der Beam-Line 6 zu einem Stoß zwischen einem Elektron des Elektronenstrahls 7 und einem in der Beam-Line 6 verbliebenen Restgasatom bzw. Restgasmolekül, so ionisiert dieses Teilchen mit einer gewissen Wahrscheinlichkeit zu einem Ion 17 (sogenanntes Rückstoßion). Ein derartiges Ion 17 hat typischerweise eine geringe kinetische Energie, welche im Bereich zwischen einigen Mikroelektronenvolt (µeV) bis hin zu einigen wenigen Millielektronenvolt (meV) reicht (typische Werte). Damit die (in aller Regel nicht übermäßig große Anzahl an) Ionen 17 möglichst vollständig gemessen werden kann, werden diese durch Anlegen eines elektrischen Feldes 18 mit geeigneter Größe und Richtung in Richtung einer Ionenkollektorplatte 19 gelenkt. Die Ionenkollektorplatte 19 weist in ihrer Mitte eine Durchgangsloch 20 für den Elektronenstrahl 7 auf. Die lonenkollektorplatte 19 ist beispielsweise mit einem kreisrunden Querschnitt und einer zylindermantelausschnittsartigen Formgebung ausgebildet. Eine typische Flugbahn eines Rückstoßions 17 ist Fig. 1 durch einen Pfeil 21 skizziert.

Das elektrische Feld 18, das dazu dient, die erzeugten Ionen 17 auf die Ionenkollektorplatte 19 zu lenken, wird durch Anlegen einer entsprechenden elektrischen Spannung zwischen der Ionenkollektorplatte 19 und der Begrenzungswand 15 der Beam-Line 6 erreicht. Typischerweise werden hier Spannungen im Bereich von einigen wenigen Volt angelegt. Lediglich der Vollständigkeit halber wird darauf hingewiesen, dass in Abhängigkeit der Geometrie von Strahlrohr und Kollektor gegebenenfalls zusätzliche Maßnahmen (wie beispielsweise Korrekturbleche, Spannungsteiler und dergleichen) nötig sind, um ein hinreichend homogenes elektrisches Feld zu erzeugen.

Aufgrund des nur schwachen elektrischen Feldes 18 wird der Elektronenstrahl 7 auf seinem Weg durch die Beam-Line 6 nur vergleichsweise geringfügig beeinflusst. Typischerweise sind die kinetischen Energien der Elektronen im Elektronenstrahl 7 im Bereich zwischen 20 eV bis 1 keV. Allein hieraus wird deutlich, dass in den meisten Fällen eine nur sehr geringe Beeinflussung der Flugbahn des Elektronenstrahls 7 erfolgt.

Die durch Stoßionisation erzeugten Ionen 17, die auf der Ionenkollektorplatte 19 gesammelt werden, werden mit Hilfe eines Ionenstrommessgerätes 22 als Ionenstrom gemessen. Durch Verhältnisbildung des Ionenstroms 22 im Verhältnis zum Elektronenstrom 14 ist es möglich, den Restgasdruck unmittelbar im Bereich der Beam-Line 6 zu bestimmen. Dies ist bei bislang kommerziell erhältlichen Ionisationsmanometern nicht möglich, da bei diesen "lediglich" der Druck in einem seitlich durch eine Flanschverbindung 12 angeflanschten Bereich gemessen wird.

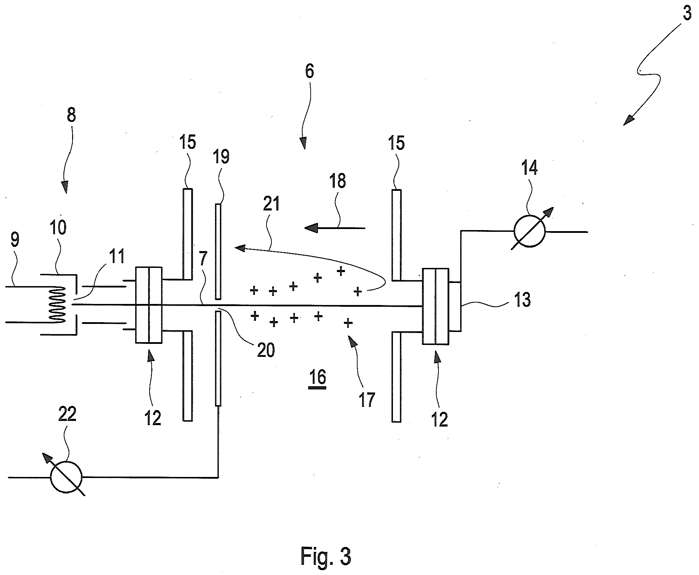

Beim in Fig. 1 dargestellte Ausführungsbeispiel wird der Ionenstrom durch ein Ionenstrommessgerät 22 gemessen. Insbesondere dann, wenn anstelle einer UHV-Umgebung 16 eine XHV-Umgebung vorliegt, kann es vorkommen, dass die Wechselwirkungsrate zwischen Elektronen und Restgasmolekülen bzw. Restgasatomen zu gering ist, um unter Verwendung eines (unmittelbar gewonnenen) Ionenstroms 22 zu sinnvollen Messergebnissen zu kommen. Hier ist es möglich, einzelne Ereignisse beispielsweise mit Hilfe eines elektronischen Rechners oder dergleichen zu registrieren. Insbesondere können dazu messverstärkende Anordnungen verwendet werden. Insbesondere können beispielsweise anstelle einer Ionenkollektorplatte 19 auch sogenannte "Channeltrons" oder "Micro channel plates" Verwendung finden (vergleiche auch Fig. 3). Bei Verwendung von messverstärkenden Anordnungen wird in der Regel kein (kontinuierlicher) Ionenstrom mehr gemessen, sondern es werden in aller Regel vielmehr einzelne Ereignisse gezählt und die Häufigkeit der einzelnen Ereignisse im zeitlichen Mittel als Messwert genutzt. Die derart gewonnene Zählrate korrespondiert in aller Regel mit dem Druck.

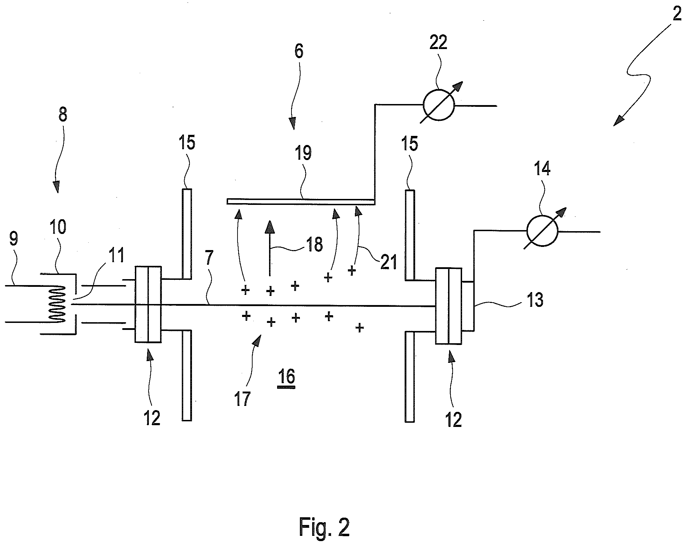

In den Fig. 2 und 3 sind Variationen des in Fig. 1 dargestellten lonisationsmanometers 1 skizziert.

Das in Fig. 2 dargestellte Ionisationsmanometer 2 bzw. das in Fig. 3 dargestellte Ionisationsmanometer 3 unterscheidet sich insbesondere durch die Lage und Anordnung der Ionenkollektorplatte 19 vom in Fig. 1 dargestellten Ionisationsmanometer 1. Korrespondierend zur Anordnung der lonenkollektorplatte 19 ist auch das elektrische Feld 18, welches die erzeugten Ionen 17 zur Ionenkollektorplatte 19 hin beschleunigt entsprechend unterschiedlich (insbesondere von der Richtung her).

Beim in Fig. 2 dargestellten zweiten Ausführungsbeispiel eines Ionisationsmanometers 2 sollte darauf hingewiesen werden, dass die lonenkollektorplatte 19 ebenfalls mit einem Durchgangsloch versehen werden kann (beispielsweise um einen in der Beam-Line 6 umlaufenden Teilchenstrahl hindurch zu lassen). Ebenso ist es aber auch möglich, dass der Teilchenstrahl beispielsweise von vorne nach hinten die Zeichenebene von Fig. 2 durchdringt (bzw. die Ionenkollektorplatte 19 entsprechend "verdreht" ist).

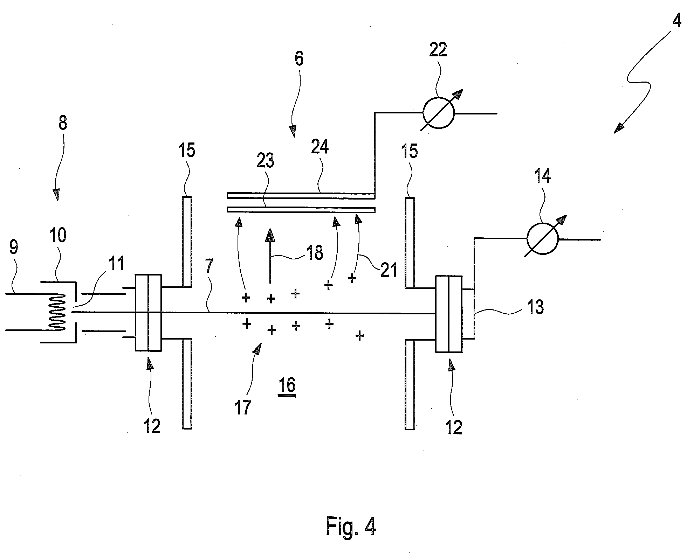

Beim in Fig. 4 dargestellten vierten Ausführungsbeispiel eines lonisationsmanometers wird anstelle einer "einfachen" Ionenkollektorplatte 19 eine Kombination aus einer Micro channel plates 23 und einem Sekundärelektronenkollektor 24 verwendet. Die Micro channel plates 23 (also solche grundsätzlich im Stand der Technik bekannt) fängt Ionen 17 ein. Wird die Micro channel plates 23 von einem Ion 17 getroffen, so erzeugt diese eine Elektronenlawine - eine sogenannte Sekundärelektronenlawine. Von der Grundidee her ähnelt die Micro channel plates 23 einem sogenannten Photomultiplier, wobei anstelle von Photonen Ionen 17 als "Eingangsstrahlung" dienen. Der derart verstärkte Ionenstrom wird von einem Sekundärelektronenkollektor 24 erfasst und über ein Ionenstrommessgerät 22 (welches eine entsprechend angepasste Empfindlichkeit benötigt und/oder beispielsweise als zählendes und/oder integrierendes und/oder als eine Zählrate registrierendes Messgerät ausgeführt ist) gemessen.

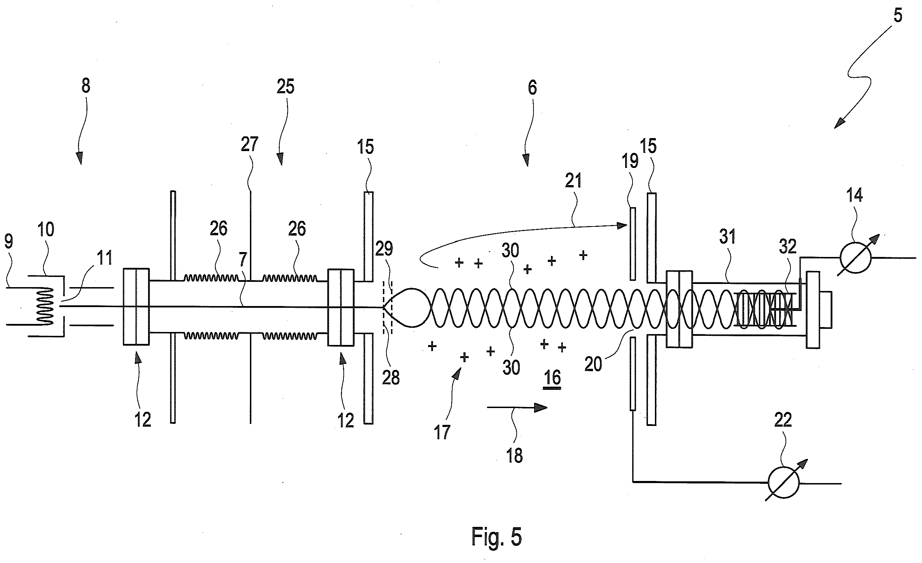

In Fig. 5 ist schließlich ein weiteres, fünftes Ausführungsbeispiel eines lonisationsmanometers 5 dargestellt. Dieses weist zusätzlich zu den in den Fig. 1 bis 4 dargestellten Ausführungsbeispielen von Ionisationsmanometern 1, 2, 3, 4 weitere Komponenten auf. Insbesondere ist in Fig. 5 eine thermische Isolationsanordnung 25 vorgesehen. Diese besteht aus zwei thermisch isolierenden Wellrohren 26. Zusätzlich ist zwischen den beiden Wellrohren 26 ein sogenanntes 77 K-Wärmeschild angeordnet, welches mit Hilfe von flüssigem Stickstoff gekühlt werden kann. Durch die thermische Isolationsanordnung 25 ist der Wärmeeintrag, der durch die Glühkathode 9 in die Beam-Line 6 hinein erfolgt, überaus gering. Das in Fig. 5 dargestellte Ionisationsmanometer 5 ist daher besonders geeignet zum Einsatz in Verbindung mit kryostatischen Aufbauten (wobei die kryostatischen Aufbauten vorliegend nicht näher dargestellt sind).

Damit der Elektronenstrahl 7 die thermische Isolationsanordnung 25 im wesentlichen verlustfrei durchfliegen kann, werden die Elektronen in diesem Bereich mit einer vergleichsweise hohen kinetischen Energie geführt. Dies würde im Bereich der Beam-Line 6 dazu führen, dass es zu einer zu geringen Anzahl an Interaktionen zwischen (schnellen) Elektronen und Restgasatomen/Restgasmolekülen kommen würden. Dadurch würden zu wenige Ionen 17 erzeugt, wodurch die Messgenauigkeit zu sehr leiden würde. Aus diesem Grund wird an der eingangsseitigen Begrenzungswand 15 der Beam-Line 6 eine Kombination aus einem Abbremsgitter 28 und einem Defokussierungsgitter 29 vorgesehen. Die Kombination aus den beiden Gittern 28, 29 bewirkt eine Auffächerung des Elektronenstrahls 7 in diesem Bereich, sodass eine Vielzahl an Elektronenbahnen 30 entsteht. Durch das Vorsehen von geeigneten magnetischen Feldern werden die Elektronenbahnen 30 auf eine angenäherte Schraubenform gezwungen. Dadurch liegen die Elektronen einerseits in einem günstigen Energiebereich (beispielsweise ca. 200 eV) vor und durchlaufen darüber hinaus eine "künstlich verlängerte" Strecke innerhalb der Beam-Line 6. Hierdurch kommt es zu einer vergleichsweise großen Anzahl an Interaktionen zwischen Elektronen und Restgasmolekülen/Restgasatomen. Die derart erzeugten Ionen 17 werden in an sich bekannter Weise durch ein elektrisches Feld 18 einer Ionenkollektorplatte 19 zugeführt und der resultierende Gesamtstrom wird über ein lonenstrommessgerät 22 gemessen.

Die Elektronen 7, 30 werden durch einen Auffangzylinder 31 mit einer gitterförmigen Zylinderelektrode 32 gesammelt. Der Elektronenstrom wird dabei erneut durch ein Elektronenstrommessgerät 14 gemessen und dient als Vergleichsnormal für den gemessenen Ionenstrom 22.

Der Vollständigkeit halber wird darauf hingewiesen, dass das fünfte Ausführungsbeispiel eines Ionisationsmanometers 5 auch mit einem einfacheren Aufbau realisiert werden kann. Insbesondere sind beispielsweise Abbremsgitter 28, magnetische Felder und/oder zyklotronartige Elektronenbahnen 30 nicht zwangsläufig erforderlich.

### Bezugszeichenliste:

- 1.: Ionisationsmanometer
- 2.: Ionisationsmanometer
- 3.: Ionisationsmanometer
- 4.: Ionisationsmanometer
- 5.: Ionisationsmanometer
- 6.: Beam-Line
- 7.: Elektronenstrahl
- 8.: Elektronenquelle
- 9.: Glühkathode
- 10.: Beschleunigungsanode
- 11.: Durchgangsloch
- 12.: Flanschverbindung
- 13.: Elektronenkollektor
- 14.: Elektronenstrommessgerät
- 15.: Begrenzungswand
- 16.: UHV-Umgebung
- 17.: Ion
- 18.: Elektrisches Feld
- 19.: Ionenkollektorplatte
- 20.: Durchgangsloch
- 21.: Flugbahn
- 22.: Ionenstrommessgerät
- 23.: Micro channel plates
- 24.: Sekundärelektronenkollektor
- 25.: thermische Isolationsanordnung
- 26.: Wellrohr
- 27.: 77 K-Wärmeschild
- 28.: Abbremsgitter
- 29.: Defokussierungsgitter
- 30.: Elektronenbahnen
- 31.: Auffangzylinder
- 32.: Zylinderelektrode

## Patentansprüche

1. Messvorrichtung (1, 2, 3, 4, 5), welche als Druckmessvorrichtung ausgebildet ist, aufweisend zumindest eine Elektronenfreisetzungsvorrichtung (8) zur Erzeugung eines Elektronenstrahls (7), zumindest eine Elektronenaufnahmevorrichtung (13, 31, 32) und zumindest eine Ionenaufnahmevorrichtung (19, 23, 24), **gekennzeichnet durch** zumindest eine Messstreckeneinrichtung (6), welche gesondert von der zumindest einen Elektronenaufnahmevorrichtung (13, 31, 32) ausgebildet ist und eine Durchgangseinrichtung für einen Teilchenstrahl aufweist, wobei im Bereich der Messstreckeneinrichtung (6) die Druckmessung **durch** Interaktion zwischen Restgasatomen und Elektronen erfolgt, wobei Ionen, welche im Bereich der Messstreckeneinrichtung (6) erzeugt werden, mit der Ionenaufnahmevorrichtung (19, 23, 24) messtechnisch erfasst werden und wobei die Durchgangseinrichtung derart ausgebildet und eingerichtet ist, dass sie den Durchgang eines Teilchenstrahls ermöglicht, und wobei die zumindest eine Elektronenfreisetzungsvorrichtung (8), die zumindest eine Elektronenaufnahmevorrichtung (13, 31, 32) und die zumindest eine Ionenaufnahmevorrichtung (19, 23, 24) räumlich voneinander getrennt ausgebildet und angeordnet sind und die Elektronenfreisetzungsvorrichtung (8) über eine Flanschverbindung (12) vakuumdicht mit einer Beam-Line (6) verbindbar ist, um den Elektronenstrahl (7) auf eine gegenüberliegende Begrenzungswand (15) der Beam-Line (6) zu richten, und die Elektronenaufnahmevorrichtung (13, 31, 32) im Zielgebiet des Elektronenstrahls (7) über eine vakuumdichte Flanschverbindung (12) verbindbar ist, so dass der Elektronenstrahl (7) **durch** das zu vermessende Gasvolumen sendbar ist und ein Teilchenstrahl durchgehen kann, so dass die Messung unmittelbar im Bereich des Teilchenstrahls durchführbar ist.

2. Messvorrichtung (1, 2, 3, 4, 5) nach Anspruch 1, **dadurch gekennzeichnet, dass** die zumindest eine Messstreckeneinrichtung (6) zusätzlich zumindest eine Transporteinrichtung für den Transport größerer Mengen Materie, zumindest eine zusätzliche Messeinrichtung, zumindest eine zusätzliche Sensoreinrichtung, zumindest eine Experimentiereinrichtung, zumindest eine Beeinflussungseinrichtung zur Beeinflussung eines Teilchenstahls oder zumindest eine Pumpeinrichtung aufweist.

3. Messvorrichtung (1, 2, 3, 4, 5) nach einem der vorangehenden Ansprüche, **dadurch gekennzeichnet, dass** die Messvorrichtung (1, 2, 3, 4, 5) zumindest teilweise als Niederdruckmessvorrichtung, bevorzugt für Tieftemperaturumgebungen ausgebildet ist.

4. Messvorrichtung (1, 2, 3, 4, 5) nach einem der vorangehenden Ansprüche, **dadurch gekennzeichnet, dass** die Messvorrichtung (1, 2, 3, 4, 5) derart ausgebildet und eingerichtet ist, dass in zumindest einem Bereich der Messstreckeneinrichtung (6) zumindest ein Teil der Elektronen (7, 30) zumindest zeitweise eine Teilchenenergie zwischen 20 eV und 1 keV, bevorzugt zwischen 50 eV und 500 eV, besonders bevorzugt zwischen 80 eV und 300 eV, insbesondere zwischen 100 eV und 200 eV aufweist.

5. Messvorrichtung (1, 2, 3, 4, 5) nach einem der vorangehenden Ansprüche, **dadurch gekennzeichnet, dass** in zumindest einem Bereich der Messstreckeneinrichtung (6) zumindest zeitweise ein Elektronenstrom (7, 30) mit einer Stromstärke von ≥ 0,2 mA, bevorzugt von ≥ 0,5 mA, besonders bevorzugt von ≥ 0,8 mA, insbesondere von ≥ 1 mA vorhanden ist.

6. Messvorrichtung (1, 2, 3, 4, 5) nach einem der vorangehenden Ansprüche, **dadurch gekennzeichnet, dass** die zumindest eine Ionenaufnahmevorrichtung (19, 23, 24) als Ionenabsaugvorrichtung oder als flächige Ionenkollektoreinrichtung ausgebildet ist.

7. Messvorrichtung (1, 2, 3, 4, 5) nach einem der vorangehenden Ansprüche, **dadurch gekennzeichnet, dass** die zumindest eine lonenaufnahmevorrichtung (19, 23, 24) zumindest bereichsweise zumindest eine Teilchenvervielfachungs-Verstärkungseinrichtung (23) aufweist.

8. Messvorrichtung (1, 2, 3, 4, 5) nach einem der vorangehenden Ansprüche, **dadurch gekennzeichnet, dass** die zumindest eine Elektronenaufnahmevorrichtung (13, 31, 32), die zumindest eine lonenaufnahmevorrichtung (19, 23, 24) und die zumindest eine Elektronenfreisetzungsvorrichtung (8) zumindest bereichsweise durch zumindest eine thermische Isolationseinrichtung (12, 26, 27) voneinander getrennt sind.

9. Messvorrichtung (1, 2, 3, 4, 5) nach einem der vorangehenden Ansprüche, **gekennzeichnet durch** zumindest eine Elektronenbeschleunigungseinrichtung (10, 11), zumindest eine Elektronenfokussiereinrichtung (11), zumindest eine Elektronenablenkeinrichtung, zumindest eine Elektronenabbremseinrichtung (28), zumindest eine Elektronendefokussierungseinrichtung (29), zumindest eine Streuelektronenabsaugeinrichtung, zumindest eine lonenabsaugeinrichtung (19, 23) oder zumindest eine Bahnbeeinflussungseinrichtung.

10. Niederdruckeinrichtung (4), insbesondere Teilchenbeschleunigereinrichtung und/oder Teilchenstrahlführungseinrichtung, umfassend zumindest eine Messvorrichtung (1, 2, 3, 4, 5) nach einem der Ansprüche 1 bis 9.

## Claims

1. A measuring device (1, 2, 3,4, 5) in the form of a pressure measuring device, comprising at least one electron releasing device (8) for producing an electron beam (7), at least one electron collecting device (13, 31, 32), and at least one ion collecting device (19, 23, 24); **characterized by**
at least one measurement line device (6) which is provided separately from the at least one electron collecting device (13, 31, 32) and which has a passage means for a particle beam, wherein in the region of the measurement line device (6) pressure measurement is accomplished via interaction between residual gas atoms and electrons, wherein ions generated in the region of the measurement line device (6) are metrologically determined by the ion collecting device (19, 23, 24), and wherein the passage means is adapted and arranged so as to allow passage of a particle beam, and wherein the at least one electron releasing device (8), the at least one electron collecting device (13, 31, 32), and the at least one ion collecting device (19, 23, 24) are configured and arranged spatially separated from each other, and wherein the electron releasing device (8) is vacuum-tightly connectable to a beam line (6) via a flange connection (12) in order to direct the electron beam (7) onto an opposing boundary wall (15) of the beam line (6), and wherein the electron collecting device (13, 31, 32) is connectable via a vacuum-tight flange connection (12) in the target area of the electron beam (7) so that the electron beam (7) can be directed through the gas volume to be measured and a particle beam can pass through so that the measurement can be performed directly in the region of the particle beam.

2. The measuring device (1, 2, 3, 4, 5) according to claim 1, **characterised in that** the at least one measurement line device (6) further comprises at least one transporting means for transporting major quantities of matter, at least one additional measuring device, at least one additional sensor means, at least one experimental device, at least one influencing means for influencing a particle beam, or at least one pumping means.

3. The measuring device (1, 2, 3, 4, 5) according to any one of the preceding claims, **characterised in that** the measuring device (1, 2, 3,4, 5) is configured at least partially as a low-pressure measuring device, preferably for low-temperature environments.

4. The measuring device (1, 2, 3, 4, 5) according to any one of the preceding claims, **characterised in that** the measuring device (1, 2, 3, 4, 5) is adapted and arranged so that in at least one region of the measurement line device (6) at least a portion of the electrons (7, 30) has a particle energy between 20 eV and 1 keV, preferably between 50 eV and 500 eV, more preferably between 80 eV and 300 eV, most preferably between 100 eV and 200 eV, at least temporarily.

5. The measuring device (1, 2, 3, 4, 5) according to any one of the preceding claims, **characterised in that** in at least one region of the measurement line device (6) an electron current (7, 30) is existing with an amperage of ≥ 0.2 mA, preferably ≥ 0.5 mA, more preferably ≥ 0.8 mA, most preferably ≥ 1 mA, at least temporarily.

6. The measuring device (1, 2, 3, 4, 5) according to any one of the preceding claims, **characterised in that** the at least one ion collecting device (19, 23, 24) is configured as an ion extraction means or as a planar ion collecting means.

7. The measuring device (1, 2, 3, 4, 5) according to any one of the preceding claims, **characterised in that** the at least one ion collecting device (19, 23, 24) includes at least one particle multiplying amplifier means (23), at least in sections thereof.

8. The measuring device (1, 2, 3, 4, 5) according to any one of the preceding claims, **characterised in that** the at least one electron collecting device (13, 31, 32), the at least one ion collecting device (19, 23, 24), and the at least one electron releasing device (8) are separated from each other by at least one thermal insulation means (12, 26, 27), at least in sections thereof.

9. The measuring device (1, 2, 3, 4, 5) according to any one of the preceding claims, **characterised by** at least one electron accelerating means (10, 1), at least one electron focusing means (11), at least one electron deflecting means, at least one electron deceleration means (28), at least one electron defocussing means (29), at least one scattered electrons extraction means, at least one ion extraction means (19, 23), or at least one path influencing means.

10. A low-pressure apparatus, in particular a particle accelerator device and/or particle beam guiding device, comprising at least one measuring device (1, 2, 3, 4, 5) according to any one of claims 1 to 9.

## Revendications

1. Dispositif de mesure (1, 2, 3, 4, 5) qui est réalisé comme dispositif de mesure de pression, présentant au moins un dispositif d'émission d'électrons (8) destiné à produire un faisceau d'électrons (7), au moins un dispositif de réception d'électrons (13, 31, 32) et au moins un dispositif de réception d'ions (19, 23, 24), **caractérisé par** au moins un dispositif à chemin de mesure (6) qui est réalisé séparément du dispositif de réception d'électrons (13, 31, 32), au nombre d'au moins un, et présente un dispositif de passage pour un faisceau de particules, sachant que dans la région du dispositif à chemin de mesure (6) la mesure de pression a lieu par interaction entre des atomes de gaz résiduel et des électrons, sachant que des ions qui sont produits dans la région du dispositif à chemin de mesure (6) sont détectés par des techniques métrologiques par le dispositif de réception d'ions (19, 23, 24), et sachant que le dispositif de passage est conçu et agencé de manière telle qu'il permet le passage d'un faisceau de particules, et sachant que le dispositif d'émission d'électrons (8), au nombre d'au moins un, le dispositif de réception d'électrons (13, 31, 32), au nombre d'au moins un, et le dispositif de réception d'ions (19, 23, 24), au nombre d'au moins un, sont réalisés et disposés séparément dans l'espace les uns par rapport aux autres, et le dispositif d'émission d'électrons (8) peut être relié par l'intermédiaire d'un raccord à brides (12) de façon étanche au vide à une ligne de faisceau (6) afin de diriger le faisceau d'électrons (7) sur une paroi de limitation (15) opposée de la ligne de faisceau (6), et le dispositif de réception d'électrons (13, 31, 32) peut être relié, dans la zone cible du faisceau d'électrons (7), par l'intermédiaire d'un raccord à brides (12) étanche au vide, de sorte que le faisceau d'électrons (7) peut être envoyé à travers le volume de gaz à mesurer et un faisceau de particules peut passer, de sorte que la mesure peut être réalisée directement dans la région du faisceau de particules.

2. Dispositif de mesure (1, 2, 3, 4, 5) selon la revendication 1, **caractérisé en ce que** le dispositif à chemin de mesure (6), au nombre d'au moins un, présente en plus au moins un dispositif de transport pour le transport de quantités relativement importantes de matières, au moins un dispositif de mesure supplémentaire, au moins un dispositif capteur supplémentaire, au moins un dispositif d'expérimentation, au moins un dispositif d'influence pour agir sur un faisceau de particules ou au moins un dispositif de pompage.

3. Dispositif de mesure (1, 2, 3, 4, 5) selon l'une des revendications précédentes, **caractérisé en ce que** le dispositif de mesure (1, 2, 3, 4, 5) est réalisé au moins en partie comme dispositif de mesure basse pression, de préférence pour des environnements à basse température.

4. Dispositif de mesure (1, 2, 3, 4, 5) selon l'une des revendications précédentes, **caractérisé en ce que** le dispositif de mesure (1, 2, 3, 4, 5) est conçu et agencé de manière à ce que, dans au moins une zone du dispositif à chemin de mesure (6), au moins une partie des électrons (7, 30) présente au moins temporairement une énergie de particules comprise entre 20 eV et 1 keV, de préférence entre 50 eV et 500 eV, de façon particulièrement avantageuse entre 80 eV et 300 eV, notamment entre 100 eV et 200 eV.

5. Dispositif de mesure (1, 2, 3, 4, 5) selon l'une des revendications précédentes, **caractérisé en ce que**, dans au moins une zone du dispositif à chemin de mesure (6), il existe au moins temporairement un flux d'électrons (7, 30) avec une intensité de courant de ≥ 0,2 mA, de préférence de ≥ 0,5 mA, de façon particulièrement avantageuse de ≥ 0,8 mA, notamment de ≥ 1 mA.

6. Dispositif de mesure (1, 2, 3, 4, 5) selon l'une des revendications précédentes, **caractérisé en ce que** le dispositif de réception d'ions (19, 23, 24), au nombre d'au moins un, est réalisé comme dispositif d'aspiration d'ions ou comme dispositif collecteur d'ions plan.

7. Dispositif de mesure (1, 2, 3, 4, 5) selon l'une des revendications précédentes, **caractérisé en ce que** le dispositif de réception d'ions (19, 23, 24), au nombre d'au moins un, présente au moins dans certaines parties au moins un dispositif amplificateur de multiplication de particules (23).

8. Dispositif de mesure (1, 2, 3, 4, 5) selon l'une des revendications précédentes, **caractérisé en ce que** le dispositif de réception (13, 31, 32), au nombre d'au moins un, le dispositif de réception d'ions (19, 23, 24), au nombre d'au moins un, et le dispositif d'émission d'électrons (8), au nombre d'au moins un, sont séparés les uns des autres, au moins dans certaines parties, par au moins un dispositif d'isolation thermique (12, 26, 27).

9. Dispositif de mesure (1, 2, 3, 4, 5) selon l'une des revendications précédentes, **caractérisé en ce qu'**il comporte au moins un dispositif d'accélération d'électrons (10, 11), au moins un dispositif de focalisation d'électrons (11), au moins un dispositif de déviation d'électrons, au moins un dispositif de freinage d'électrons (28), au moins un dispositif de défocalisation d'électrons (29), au moins un dispositif d'aspiration d'électrons dispersés, au moins un dispositif d'aspiration d'ions (19, 23) ou au moins un dispositif d'influence de trajectoire.

10. Dispositif à basse pression (4), en particulier dispositif accélérateur de particules et/ou dispositif de guidage de faisceau de particules, comprenant au moins un dispositif (1, 2, 3, 4, 5) selon l'une des revendications 1 à 9.
